# EUROPEAN PATENT APPLICATION

(11) **EP 4 667 046 A1**
(43) Date of publication of application: **24.12.2025**
(21) Application number: 24223772.5
(22) Date of filing: 30.12.2024
(51) Int. Cl.: A61N 1/375, H01R 24/00, H01R 43/00, H01R 4/50, H01R 9/05, H01R 11/11, A61N 1/30, A61N 1/378, A61N 1/39, A61N 1/05

(54) **CONNECTOR FOR ELECTRICALLY CONNECTING AN IMPLANTABLE LEAD DEVICE**

(30) Priority: 18.06.2024 EP 24182934
(71) Applicant: ODU GmbH & Co KG., 84453 Mühldorf a. Inn (DE)
(72) Inventor: SCHILDHAUER, Moritz, 84453 Muehldorf a. Inn (DE); SCHABERL, Josef, 84453 Muehldorf a. Inn (DE)
(74) Representative: Huebner, Stefan Rolf

(57) **Abstract**

A connector (1) for electrically connecting a proximal end of a lead device (2) that is implantable within the body of a patient and comprises a lead (3, 5), wherein the connector (1) comprises a lead contact (15, 16) for, directly or indirectly, electrically contacting, at the proximal end of the lead device (2), an electrode (8) of the lead (3, 5). The first lead contact (15, 16) comprises a lamella basket. A lead clamping fixture comprises a collet (20, 27) that can embrace the lead (3, 5) at a proximal end of the lead (3, 5) and has an external taper, and the lead clamping fixture moreover comprises a collet sleeve (21, 28) that has an internal taper for cooperating with the external taper of the collet (20, 27) to secure the collet (20, 27) on the lead (3, 5) by compressing the collet (20, 27) if the collet's (20, 27) external taper is pressed against the collet sleeve's (21, 28) internal taper.

## Description

### Field of the invention

The invention relates to a connector for electrically connecting a proximal end of a lead device that is implantable within the body of a patient.

### Background of the invention

Lead device implantable within the body of a patient, such as leads for cardiac pacemakers, are often supplied with only a very simple surgical tool. This may, for example, be a plastic sleeve which is pushed over the proximal end of the lead device. Such sleeve typically has two recesses in which the electrodes of the lead are accessible to be contacted with alligator clips. As the surgeon moves the distal end of the lead device in the patient's body, the surgeon can, with a lead placement testing apparatus attached to the crocodile clips, check whether the distal end of the lead device is in the correct position inside the patient's body. In the case of a cardiac pacemaker, for example, incorrect positioning of a lead may have severe consequences such as an increased risk of cardiac insufficiency. Once the surgeon has found the correct position of the distal end, for example in a cardiac muscle, a fixation structure, normally a wire helix, must be deployed into the patient's tissue. This typically requires rotating a lead of the lead device at the proximal end of the lead device, which in turn requires that the lead placement testing device's alligator clips and possibly even the plastic sleeve be removed.

The removal of the alligator clips and the sleeve may cause a displacement of the distal end of the lead device, which, due to the lead placement testing apparatus being disconnected, may go unnoticed. Only once the surgeon has fixed the distal end of the lead device to the patient's tissue, can the surgeon reattach the plastic sleeve and the alligator tips clips to check if the distal end is still in the correct place. If not, cumbersome removal, repositioning and testing steps are required until the distal end of the lead device is confirmed to be in the correct position. Accordingly, there is need for a tool that allows for a procedure that is more efficient and more effective in the sense that correct placement can be achieved on the first attempt and repositioning procedures as well as suboptimal final positioning can be avoided.

Moreover, a surgical method referred to as left bundle branch pacing (LBBP) involves the deployment of a lead device deep inside the septum. To achieves this, typically the outer electrode of a coaxial two electrode lead device is rotated rapidly and repeatedly. Accordingly, there is a need for means of applying such rotation in a controlled and effective manner.

US 7,753,696 B2 discloses an apparatus for facilitating the installation of an implantable lead into a subject. The apparatus includes a housing and a thumb screw for mechanically engaging a terminal pin of the lead with housing so that when the housing is rotated, the terminal pin is caused to rotate as well. Moreover, a terminal ring of the lead is contacted with an electrical contact, which in turn is contacted with an alligator clip.

From US 8,958,878 B2 an adapter body is known that releasably connects to the electrodes of a pacing lead connector to allow continuous ECG recording while manipulating the pacing lead. The adapter body has clip contacts to contacts the electrodes, including a tip electrode. Moreover, a small torque wrench can rotate the tip electrode to screw a distal tip electrode of lead into the patient's heart.

EP 2 519 310 B1 discloses an implant tool for use with an implantable lead. The implant tool has a distal clamping section to receive a terminal end of the implantable lead, and a plurality of spring contact members to engage a terminal pin and one or more ring contacts of the implantable lead. Each spring contact member is configured to receive an alligator clip to electrically connect the conductors of a Pacing System Analyzer. The implant tool also has a rotatable knob mechanism which can rotatably engage the terminal pin of the implantable lead so that a fixation helix of the implantable lead can be actuated and deployed by rotating only the knob while the implant tool and the implantable lead are held stationary.

DE 20 2023 000 413 U1 discusses improvements to a connector for implanting a lead of a cardiac pacemaker. A thumb screw is used to fix the lead to a rotation mechanism of the connector. A rotatable knob can rotate a spiral of the lead to screw the lead into a patient's cardiac muscle. Moreover, it is suggested that the connector comprise a "clamp construction" to electrically contact the lead in order to provide for a wall contact measurement of the lead independently of the position of the lead.

### Object of the invention

It is an object of the present invention to provide an improved connector for electrically connecting a proximal end of a lead device that is implantable within the body of a patient.

### Solution according to the invention

In the following, any reference to one (including the articles "a" and "the"), two or another number of objects is, provided nothing else is expressly mentioned, meant to be understood as not excluding the presence of further such objects in the invention. The reference numerals in the patent claims are not meant to be limiting but merely serve to improve readability of the claims.

According to a first aspect of the invention, the problem is solved by a connector for electrically connecting a proximal end of a lead device that is implantable within the body of a patient, the connector having the features of claim 1. The connector comprises a first lead contact for, directly or indirectly, electrically contacting, at the proximal end of the lead device, an electrode of a first lead of the lead device. The first lead contact comprises a lamella basket.

In the context of the present invention, with regard to the lead device and its component leads, the "proximal end" is the end that is to be connected to or is at least closest to the connector. Accordingly, the "distal end" is the other end, typically, but not necessarily in all embodiments, to be electrically connected to a tissue of the human body.

The terms "first" and "second" as used herein with regard to components including leads of the lead device and parts of the connector, provided nothing else is expressly mentioned, merely serve to unambiguously name such components given that in some embodiments there may be two distinct components of the same kind, then referred to as the "first" and second". If reference is made herein to a component without the indication "first" or "second" even though elsewhere herein it is differentiated between a first and a second of this kind of component, such reference is meant to refer to both the first and the second component in the sense of "the first and/or the second" component.

In the context of the present invention, a reference to a lead contact "indirectly" electrically contacting a lead electrode means that electricity is conveyed from the first lead electrode to the first lead contact via one or more intermediate conductors, for example a lead clamping fixture.

As used herein, the term "lamella basket" refers to a device that comprises multiple elastically resilient electrically conducting lamellae arranged around a longitudinal axis of the lamella basket and extending generally in parallel to the longitudinal axis in a way that each lamella can resiliently press against an electrode that is provided inside the lamella basket at the position of the longitudinal axis, thereby electrically contacting the electrode. It is an achievable advantage of a lamella basket that it can securely contact the electrode even if it is rotated. This exploits, ia, that due to the multiple lamellae, if, as a result to the electrode's rotation, the contact of one or more of the lamellae occasionally weakens or is interrupted, electricity can still be flow via the remaining lamellae.

It is an achievable advantage of this aspect of the present invention that the lamella basket can make more secure electrical contact with the electrode of a lead or an electrode of the intermediate conductor even if the electrode is rotating relatively to the lamella basket.

According to a second aspect of the invention, the problem is solved by a connector for electrically connecting a proximal end of a lead device that is implantable within the body of a patient, the connector having the features of claim 11. The connector comprises a first lead clamping fixture for clamping the first lead. The first lead clamping fixture comprises a collet that can embrace the first lead at a proximal end of the first lead and has an external taper. The first lead clamping fixture moreover comprises a collet sleeve that has an internal taper for cooperating with the external taper of the collet to secure the collet on the first lead by compressing the collet if the collet's external taper is pressed against the collet sleeve's internal taper.

In the context of the present invention, the terms "collet" and "collet sleeve" do not imply that the collet or collet sleeve has a thread as is present in many collet clamp systems. While in some embodiments the collet and the collet sleeve may have matching threads to press the collet against the collet sleeve's internal taper, in other embodiments such pressing is achieved by other means as detailed further below.

According to a third aspect of the invention, the problem is solved by a connector for electrically connecting the proximal end of a lead device that is implantable within the body of a patient, the connector having the features of claim 23. The lead device is a coaxial lead device with an outer lead and an inner lead that extends coaxially insider the outer lead and the inner lead being rotatable relatively to the outer lead. The connector comprises a first lead clamping fixture for clamping a first lead, the first lead being one lead from the group of leads comprising the inner lead and the outer lead, and a first lead handle so that a rotating of the first lead handle relatively to the housing rotates the first lead clamping fixture relatively to the housing. The connector moreover comprises a second lead clamping fixture for clamping a second lead, the second lead being the other lead from the group of leads comprising the inner lead and the outer lead, and a second lead handle so that a rotating of the second lead handle relatively to the housing rotates the second lead clamping fixture relatively to the housing.

Whenever reference is made herein to "one" component and the "other" component of a group of two components, the "one" component can be any one of the two components, and the other component is the component that is not the one component. Accordingly, the expression "one lead" refers to one lead from the group of leads comprising the inner lead and the outer lead. Thus, the present invention encompasses embodiments where the one lead is the inner lead as well as embodiments in which the one lead is the outer lead. Moreover, the expression "other lead" refers to the lead of the group of components comprising the inner lead and the outer lead that is not the one lead. Accordingly, if the one lead is the inner lead, the other lead is the outer lead and vice versa.

### Preferred embodiments of the invention

Preferred features of the invention which may be applied alone or in combination are discussed in the following and in the dependent claims.

In a preferred embodiment of the invention, the first lead is rotatable relatively to the first lead contact. Similarly, the second lead preferably is rotatable relatively to the first lead contact. Thereby it can be avoided the lead contact must be rotated when its corresponding lead rotates. This is of advantage in particular in embodiments in which the lead contact is connectable, for example via a strand of a cable, with another apparatus that cannot be rotated but is stationary such as a conventional lead placement testing apparatus.

It is preferred that the first lead contact and/or the second lead contact, more preferably both, comprise a lamella basket; more preferably one of them or both consist of a lamella basket. With this embodiment of the invention, a more secure electrical contact of the lead contact with the electrode of the first lead is achievable even in situations where the electrode of the first lead is rotating relatively to the lamella basket. This exploits, ia, that due to the multiple lamellae, if, as a result of the electrode's rotation, the contact of one or more of the lamellae occasionally weakens or is interrupted, electricity can still be flow via the remaining lamellae. The preferred lamella basket comprises 2 or more lamellae, more preferably 3 or more lamellae, even more preferably 4 or more, even more preferably 6 or more lamellae.

Preferably, the lamella basket has an essentially tubular shape, preferably with a circular cross-section. The preferred lamellae are at least locally bent towards the centre of the inner volume of the lamella basket. When the electrode is inside the lamella basket, the electrode can deflect the lamellae, preferably resiliently, radially away from the centre of the lamella basket, thereby resiliently pressing against the electrode to make electrical contact.

In some embodiments of the invention, the lamella basket is a slotted sleeve as for example offered by ODU GmbH & Co KG under the brand name TURNTACO, where parts of the sleeve between the slots can resiliently contact a corresponding electrode of a matching lead. Some or all of the slots typically extend in parallel to each other. In other embodiments, the lamella basket is for example of sheet metal, preferably with the ends of the sheet joined or at least approximated to form ring, or metal wires, preferably arrange in a circle. The preferred lamellae extend from a ring, preferably a conductive ring to which the lamellae are conductively attached, the longitudinal axis of the ring preferably coinciding with or at least extending in the same direction as the longitudinal axis of the lamella basket. In some embodiments of the lamella basket, the lamellae have open ends thus forming tongues that preferably extend essentially parallel to each other away from the ring. In other embodiments of the lamella basket, the lamellae extend, again preferably essentially parallel to each other, between two such rings. Example of lamella baskets are disclosed in EP 2015403 B1, EP 3641068 B1, EP 2209167 B1 or EP 3761455 A1 and offered by ODU GmbH & Co KG under the brand name LAMTACO.

The lamella basket can be one-sided in the sense that the lamellae at one end are fixed relatively to each other, which the other end is open, ie, free to move. Alternatively, the lamella basket can be two-sided in the sense that the lamellae at both ends are fixed relatively to each other. Ways of fixing the lamellae are for example disclosed in EP 2209167 A1, the relevant parts of which are herewith incorporated by reference into the present disclosure. For example, the fixed ends of the lamellae may join into a metal strip on one (one-sided) or both (two-sided) ends of the lamellae. Preferably, the lamellae are integral with the ring(s).

The lamella basket may be formed for example by stamping, cutting, deep drawing (as for example disclosed in EP 3761455 A1) or a combination of these methods.

The preferred lamella basket has at least one constriction for contacting the electrode of the lead when the latter is present in the inner volume. This constriction preferably is formed by the contact lamellae arched towards the axis of the contact element. In the case of a one-sided lamella basket, the constriction typically is near the open ends of the lamellae, while in a two-sided lamella basket, they are typically near the centre of the longitudinal extension of the lamella basket.

The lamella basket can, eg, be created from a generally rectangular sheet of metal by forming multiple slots, eg by stamping, to define multiple parallel, longitudinally extending lamellae which are jointed at opposite ends to the inward sides of two transversally extending webs. The sheet is then formed into a cylinder with the webs forming rings and the lamellae extending essentially parallel to the cylinder axis but being arched towards the cylinder axis.

In a preferred embodiment of the invention, via the first lead contact, the electrode of the first lead is electrically connected with a strand of a cable. Likewise, in a preferred embodiment of the invention, via the second lead contact, the electrode of the second lead is electrically connected with a strand of a cable, which strand preferably is different from the strand the first electrode is electrically connected with. In these embodiments, advantageously, electricity, including electrical signals, can be transmitted between an apparatus attached to the other end of the cable to the first and/or second lead. For example, in the case where the lead device is that of a cardiac pacemaker and/or a cardiac defibrillator, the apparatus may be a lead placement testing apparatus.

A preferred connector comprises a housing. A preferred housing comprises a housing handle for an operator of the connector to hold the connector with a hand of the operator. Preferably, an end of the cable feeds into the housing and is attached to the housing, preferably at an end of the housing handle. Preferably, at least part of the first lead contact, more preferably the entire first lead contact, is attached to the housing of the connector in a way that the first lead contact cannot rotate about its longitudinal axis. Similarly, the at least part of the preferred second lead contact, more preferably the entire second lead contact, is attached to the housing of the connector in a way that the second lead contact cannot rotate about its longitudinal axis. Thereby advantageously, the strand of the cable can be connected to the lead contact's non-rotating part, thereby simplifying the design of the connector. Preferably, the lamella basket of the lead contact is fixedly attached to the housing, more preferably at one or two rings of the lamella basket.

In a preferred embodiment of the invention, the connector comprises a first lead clamping fixture for clamping the first lead. Likewise, in a preferred embodiment of the invention, the connector comprises a second lead clamping fixture for clamping the second lead. Advantageously, the lead clamping fixture can clamp its respective lead so that the lead cannot rotate relatively to the lead clamping fixture. As a result, it is achievable with these embodiments of the invention that the lead can be rotated by rotating its respective lead clamping fixture.

Preferably, the first lead clamping fixture is rotatable relatively to the housing. Likewise, the preferred second lead clamping fixture is rotatable relatively to the housing. Thereby, advantageously, the rotating the lead clamping fixture rotates its respective lead relatively to the housing.

In some embodiments of the invention, a lead, preferably the inner lead, is provided with a fixation structure at its distal end for fixing the distal end in a tissue of the patient. A preferred fixation structure is a helix or wire. By rotating the lead, the fixation structure can be deployed into the patient's tissue, for example the patient's myocardium.

A preferred first lead clamping fixture comprises a first lead handle so that a rotating of the first lead handle relatively to the housing rotates the first lead clamping fixture relatively to the housing. Likewise, a preferred second lead clamping fixture comprises a second lead handle so that a rotating of the second lead handle relatively to the housing rotates the second lead clamping fixture relatively to the housing. Preferably, for this purpose the handle is attached to its respective clamping fixture such that the handle cannot rotate relatively to the clamping fixture. It is an achievable advantage of this embodiment of the invention that by rotating the handle relatively to the housing, an operator of the connector can rotate the respective lead.

Preferably, the first lead clamping fixture comprises a first lead screw for clamping the first lead to the first lead clamping fixture by operating the first lead screw. Likewise, preferably, the second lead clamping fixture comprises a second lead screw for clamping the second lead to the second lead clamping fixture by operating the second lead screw. The preferred first or second lead screw is arranged on or in the corresponding first or second lead handle. Particularly preferably, the lead screw and the corresponding lead handle are rotatable about the same axis.

In some embodiments of the invention, the first lead contact is directly electrically contacting the first lead electrode; in other embodiments, the first lead contact is only indirectly electrically contacting the first lead electrode. Likewise, in some embodiments of the invention, the second lead contact is directly electrically contacting the first lead electrode; in other embodiments, the second lead contact is only indirectly electrically contacting the first lead electrode.

If a lead contact electrically contacts its corresponding electrode only indirectly, the intermediate conductor in direct electrical contact with the lead contact preferably is rotatable relatively to the lead contact. Particularly preferably, the intermediate conductor is attached to the lead in a way that it cannot rotate about the lead's longitudinal axis. Most preferably, the intermediate conductor is the lead clamping fixture.

A preferred lead clamping fixture, particularly preferably one that acts as an intermediate conductor, is electrically conductive. It preferably can electrically contact the electrode of its corresponding lead when attached to an electrode of the first lead. Preferably, an outside of the lead clamping fixture serves as an electrode for the lead contact to make electrical contact, more preferably for the lamellae of the lamella basket to resiliently press against in order to make electrical contact.

Preferably, the lead contact for contacting the electrode of the inner lead is only indirectly contacting this lead electrode. With this embodiment of the invention, it is achievable that the section of the inner lead that is accessible for clamping can be used for clamping without regard for reserving some of this section for electrically contacting the inner lead; using a larger section of the inner lead for clamping can provide for a more secure fixing of the inner lead to its respective clamping fixture. Preferably, the lead contact for contacting the electrode of the outer lead is directly contacting this electrode. This can simplify the design of the connector. It exploits that, typically, a sufficiently long section of the outer electrode is accessible for clamping to allow for securely fixing the outer lead to its respective clamping fixture.

Another preferred lead clamping fixture clamps the outer lead of the lead device, preferably by clamping a cover or an insulation of outer lead, which cover or insulation, also preferably, servers a cover or an insulation of the lead device as a whole.

The preferred lead clamping fixture comprises a collet that can embrace the corresponding lead at a proximal end of the lead. The preferred lead clamping fixture moreover comprises a collet sleeve to secure the collet on the first lead. Preferably, the collet sleeve can be put over the collet. The preferred collet sleeve has an internal taper for cooperating with the collet to secure the collet on the first lead by compressing the collet if the collet is pressed against the collet sleeve's internal taper. In some embodiments, the collet moreover has an external taper to cooperate with the collet sleeve's internal taper to compress the collet. Preferably, if a lead contact electrically contacts its corresponding electrode via a lead clamping fixture comprising a collet and a collet sleeve, an outside of the collet sleeve serves as the electrode for the lamellae of the lamella basket to resiliently press against in order to make electrical contact.

A preferred lead clamping fixture comprises a lead screw that is operatively connected with one element of the group comprising the collet and the collet sleeve and comprises a thread. Moreover, a preferred lead clamping fixture comprises a lead handle that is operatively connected with the other element of the group comprising the collet and the collet sleeve and comprises a matching thread. Preferably, the thread of the lead screw runs in the tread of the lead handle so that displacement of the first lead handle relatively to the first lead screw when rotating the two handles relatively to each other can press the collet against the collet sleeve's internal taper. Thereby, it is achievable that an operator of the connector can clamp the lead clamping fixture to its corresponding lead so that the lead cannot rotate relatively to the clamping fixture and the lead can be rotated by rotating the lead clamping fixture. Moreover, it is achievable to fix the lead handle to its corresponding clamping fixture such that the handle cannot rotate relatively to the clamping fixture. As a result, in can be achieved that a rotating of the first lead handle relatively to the housing rotates the first lead clamping fixture relatively to the housing.

Preferably, the second lead clamping fixture or the first lead clamping fixture comprises a central passage through which the lead device can pass inside the connector to connect the connector to the proximal end of the lead device. Also, preferably the first lead clamping fixture or the second lead clamping fixture, more preferably a lead clamping fixture that lacks the central passage of the lead device, comprises a central passage through which a guide of the lead device can be funnelled into the lead device from the outside of the connector when the connector is connected to the proximal end of the lead device.

A preferred lead device comprises two leads. More preferably, the lead device is a coaxial lead device with an outer lead and an inner lead that extends coaxially insider the outer lead. The preferred inner lead is rotatable relatively to the outer lead. The preferred outer lead is tubular for the inner lead extend inside the outer lead. Preferably, the inner lead is rotatable relatively to the outer lead. Preferably, the first lead is the inner lead. Preferably the second lead is the outer lead.

A preferred connector comprises two lead clamping fixtures. The preferred first lead clamping fixture can be rotated independently of the second lead clamping structure. Preferably, the first and the second lead clamping fixtures are arranged coaxially, ie, they can rotate about the same axis. Preferably, all lead handles and lead screws are arranged coaxially, ie, they can be rotated about the same axis. The housing preferably is arranged between the first and the second lead handles, and the lead handles preferably are arranged on opposite ends of the housing. The housing handle preferably extends essentially perpendicularly to the common axis of the lead handles. The cable preferably enters the housing through the end of the housing handle.

### Brief description of the drawings

In the following, further preferred embodiments of invention are illustrated by means of examples. The invention is not limited to these examples, however.

The drawings schematically show:
Figure 1 A perspective view of the proximal end of a coaxial lead device that is implantable within the body of a patient with an outer lead and an inner lead;
Figure 2 A perspective view of the distal end of the coaxial lead device of Figure 1;
Figure 3 A perspective view of the proximal end of the coaxial lead device of Figures 1 and 2 with a guidewire passing through the inner lead;
Figure 4 A cross-sectional view of a connector for electrically connecting the proximal end of the lead device of Figures 1 to 3;
Figure 5 A perspective view of the connector of Figure 4;
Figure 6 A cross-sectional view of the connector of Figures 4 and 5 attached to the lead device with a guide wire of Figure 3; and
Figure 7 A perspective view of the connector of Figures 4 to 6 attached to the lead device with a guide wire of Figure 3.
Figure 8 A perspective view of the inner lead contact of the connector of Figures 4 to 7.

### Detailed description of an embodiment of the invention

In the following description of preferred embodiments of the invention, identical reference numerals refer to identical or similar components.

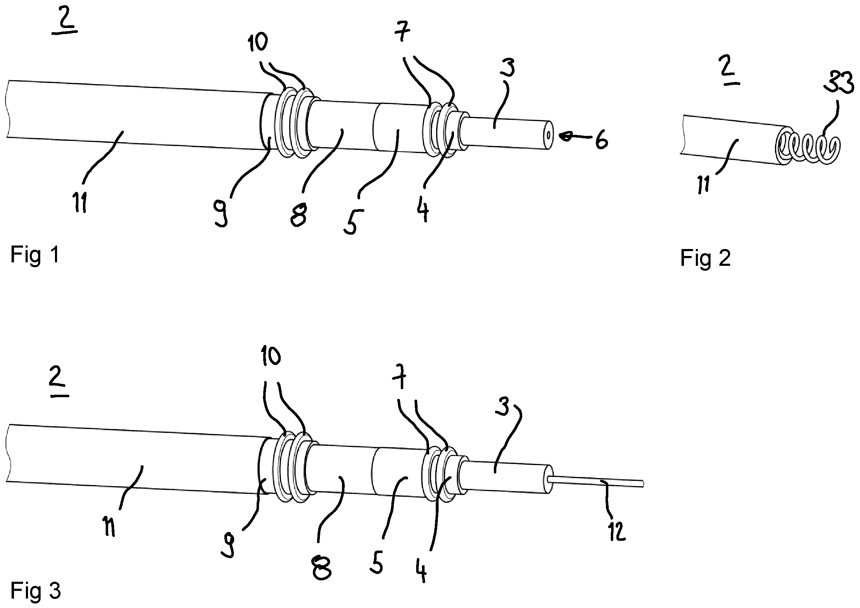

The connector 1 can be connected with the proximal end of an exemplary lead device 2 shown in **Figure 1****.** The lead device 2 is for a cardiac pacemaker and can be implanted in a vein of the patient. It has a tubular inner lead 3, the proximal end of which is exposed and serves as the electrode of the inner lead. The inner lead 3 is surrounded by an inner insulator 4 for electrical insulation against an outer lead 5. The inner lead moreover has a lumen 6. Inner seals 7 are provided to avoid contamination of the electrode of the inner lead 3 and the pacemaker, once the lead device 2 has been attached to the pacemaker and implanted into the patient's body.

The insulation 4 of the inner lead is surrounded by the tubular outer lead 5, which bears an electrode 8 of the outer lead 5. Like in the case of the inner lead 3, the outer lead 5 is surrounded by an outer insulator 9 for electrical insulation against the outside. Outer seals 10 are provided which avoid contamination of the electrode 8 of the outer lead 5; they also contribute to avoiding contamination of the inner lead 3 electrode and the pacemaker, once the lead device 2 has been attached to the pacemaker and implanted into the patient's body. A lead device cover 11 is wrapped over the outer insulation.

The distal end of the lead device of **Figure 1** is shown in **Figure 2****.** It is provided with an electrically conductive fixation helix 33 which is attached to the distal end of the inner lead 3 and can be deployed into the patient's myocardium in a screw-like fashion by means of rotating it about the longitudinal axis of the lead device 2. This can be achieved by rotating the proximal end of the inner lead 3, which is exposed at the proximal end of the lead device 2.

A guide wire 12 can be introduced into the lumen 6 of the inner lead 3 of the lead device 2. In **Figure 3****,** a section of the guide wire 12 that extends into the proximal end of the inner lead 3 is shown. The guide wire can increase the rigidity of the lead device 2. If the guide wire 12 is bent, it can moreover be used to influence and control the bending of the lead device 2.

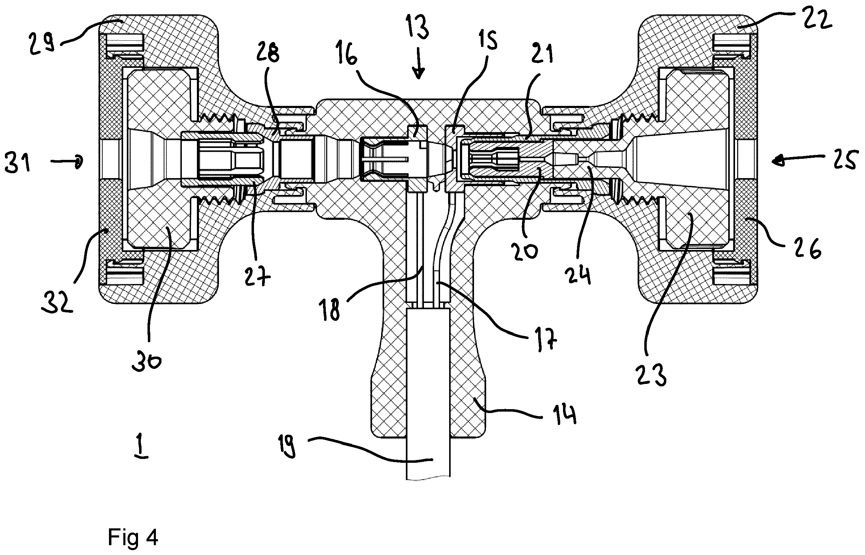

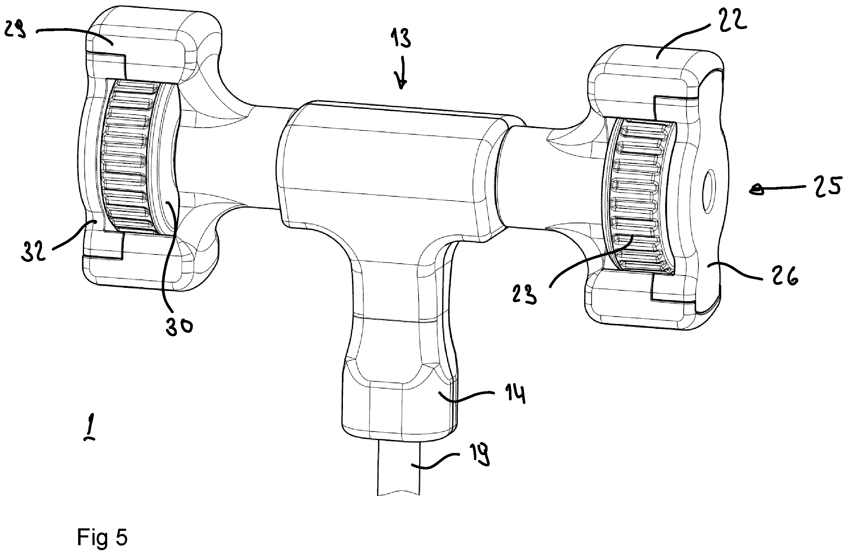

A cross-section of the connector 1 for electrically connecting the lead device 2 is shown in **Figure 4****.** It comprises a T-shaped housing 13 assembled of two shell halves as can be best seen in **Figure 5****.** The stem of the housing's T-shape servers as a housing handle 14, at which an operator of the connector 1 can grab the connector 1.

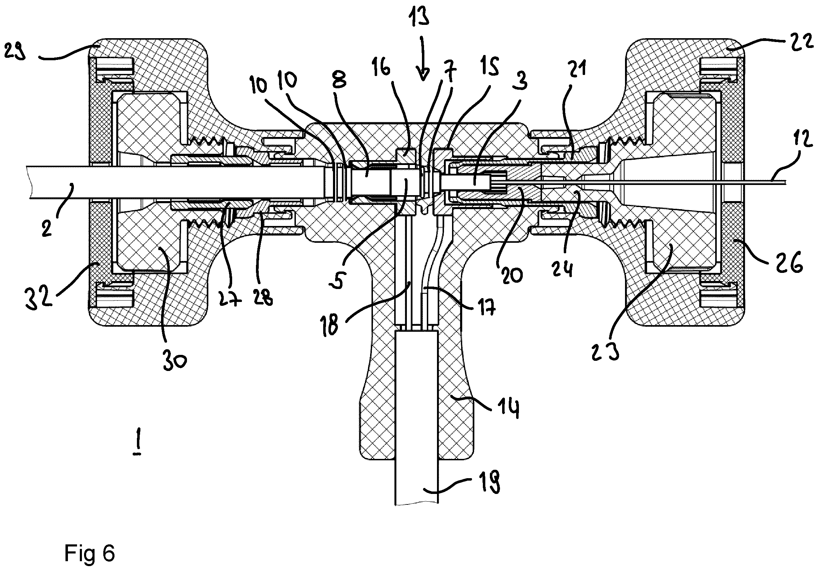

Inside the housing 13, the connector 1 comprises a first, inner lead contact 15 (on the right side of **Figure 4****)** for indirectly - via an electrode of a first, inner lead clamping fixture - contacting the electrode of the inner lead 3, and a second, outer lead contact 16 (on the left side of **Figure 4****)** for directly contacting the electrode 8 of the outer lead 5. Both lead contacts 15, 16 are lamella baskets comprising of a circular ring from which six lamellae extend. When the electrodes of the inner and the outer lead 3, 5 are inside the lamella basket, the lamellae are resiliently deflected by the electrodes, thereby resiliently pressing against the electrodes to make electrical contact. This can be seen in **Figure 6****,** which shows the connector 1 attached to the lead device 2. The lead contact's 15, 16 lamellae ensure a secure electrical contact with the electrodes of the leads 3, 5 even if the leads are rotated. At its ring, each lead contact 15, 16 is soldered to a strand 17, 18 of a two-stranded cable 19, which is led out at the bottom of the housing handle 14. At the remote end of the cable 19, the strands 17, 18 are attached to a lead placement testing apparatus.

**Figures 4** also shows a collet 20 and a collet sleeve 21 of the inner lead clamping fixture for clamping the inner lead 3. As can be seen in **Figure 6****,** the collet 20 embraces the exposed end of the inner lead 3. For clamping the inner lead 3, the collet 20 is provided with an external taper, which cooperates with an internal taper of the collet sleeve 21. If the collet's 20 external taper is pressed against the collet sleeve's 21 internal taper, the collet 20 is compressed, thereby pressing on the inner lead 3 to clamp it.

The inner lead 3 clamping fixture comprises an inner lead handle 22 and an inner lead screw 23, which in combination, operate the collet mechanism comprising the collet 20 and the collet sleeve 21. The inner lead handle 22 is attached, by means of a latch mechanism, to the right end of the T-bar of the housing 13 in a manner that it can be rotated about the longitudinal axis of the inner lead clamping mechanism, which is also the longitudinal axis of the lead device 2 when connected to the connector 1. The latch mechanism allows it to be snapped onto the housing during assembly of the connector. The part of the inner lead handle 22 adjacent to the housing 13 is tubular and the collet sleeve 21 extends from the tubular part of the inner lead handle 22 into the housing 13. The outside diameter of the collet sleeve 21 expands in the tubular part of the inner lead handle 22 to create a ring-shaped step which sits on a corresponding ring shaped step inside the tubular part of the inner lead handle 22, so that collet sleeve 21 cannot slip out of the inner lead handle 22 in the direction of the housing 13. The tubular part of the inner lead handle 22 moreover comprises an inner thread in which an outer thread of the inner lead screw 23 runs so that the latter can be tightened and loosened in the direction of the longitudinal axis of the inner lead clamping mechanism by rotating it about this axis. The head of the inner lead screw 23 is accessible for an operator through windows in the inner lead handle 22. Moreover, as the inner lead screw 23 is tightened, a plunger 24 of the inner lead screw 23 presses on the collet 20 to press the collet's 20 external taper against the collet sleeve's 21 internal taper.

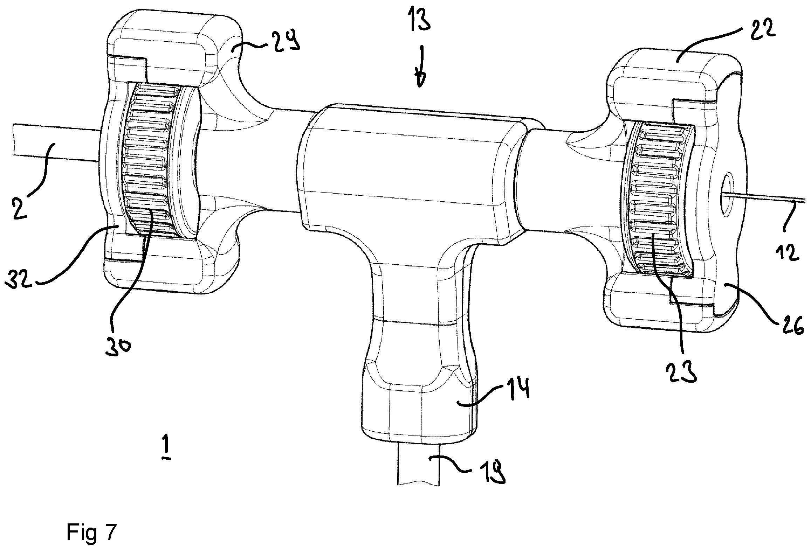

The inner lead clamping fixture comprises a central passage 25 through which the guide wire 12 of lead device 2 can be introduced into the connector 1. The central passage 25 is formed by an aperture in a lid 26 that closes off the inner lead handle 22, followed by channel that extends through the inner lead screw 23 with several inner tapers, and the collet 20, likewise with an inner taper to funnel the lead wire 12 into the lumen 6 of the tubular inner lead 3. **Figures 6** and **7** show how the guide wire 12 passes through the central passage 25 to extend further into the lumen 6 of the inner lead 3.

In the example of the connector 1 shown in **Figures 4** to **7****,** a second, outer lead clamping fixture is attached to an end of the housing 13 opposite the end where the inner lead clamping fixture is attached. The outer lead 5 can be rotated independently of the rotation of the inner lead 3. The second, outer lead clamping fixture largely resembles the first, inner lead fixture. A collet 27 of the outer lead clamping fixture embraces a section of the proximal end of the cover 11 of the outer lead 5. For clamping the outer lead 5, the collet 27 is provided with an external taper, which cooperates with an internal taper of a collet sleeve 28. If the collet's 27 external taper is pressed against the collet sleeve's 28 internal taper, the collet 27 is compressed, thereby pressing on the outer lead 5, via the cover 11 and the insulation 9, to clamp it.

The outer lead clamping fixture moreover comprises an outer lead handle 29 and an outer lead screw 30, which in combination, operate the collet mechanism comprising the collet 27 and the collet sleeve 28. The outer lead handle 29 is attached, by means of a latch mechanism, to the left end of the T-bar of the housing 13 in a manner that in can be rotated about the longitudinal axis of the outer lead clamping mechanism, which is also the longitudinal axis of the lead device 2 when connected to the connector. The latch mechanism allows it to be snapped onto the housing 13 during assembly of the connector 1. The part of the outer lead handle 29 adjacent to the housing 13 is tubular and accommodates the collet sleeve 28. The outside diameter of the collet sleeve 28 expands to create a ring-shaped step which sits on a corresponding ring-shaped step inside the tubular part of the of the outer lead handle 29 so that collet sleeve 28 cannot slip out of the outer lead handle 29 in the direction of the housing 13. The tubular part of the outer lead handle 29 moreover comprises an inner thread in which an outer thread of the outer lead screw 30 runs so that the latter can be tightened and loosened in the direction of the longitudinal axis of the outer lead clamping mechanism by rotating it about this axis. The head of the outer lead screw 30 is accessible for an operator through windows in the outer lead handle 29. The collet 27 sits in the shaft of the outer lead screw 30 such that, as the outer lead screw 30 is tightened, the outer lead screw 30 presses on the collet 27 to press the collet's 27 outer taper against the collet sleeve's 28 inner taper.

The outer lead clamping fixture comprises a central passage 31 through which the lead device 2 can be introduced into connector 1. The central passage 31 is formed by an aperture in a lid 32 that closes off the outer lead handle 29, followed by channel that extends through the outer lead screw 30 which comprises inner tapers, the collet 27, and an extension of the collet sleeve 28. Figures 6 and 7 show how the lead device 2 passes through the central passage 31. The central passage 31 is shaped to provide sufficient space for the seals 7, 10 of the lead device to not be damages during insertion.

For connecting the connector 1 to the lead device 2, the lead device 2 is first inserted into the connector 1 from the left side without the guide wire 12. The outer lead screw 30 and the inner lead screw 23 are then tightened, and the guide wire 12 is inserted if required. The outer lead 5 and the inner lead 3 of the lead device 2 are now reliably electrically connected to the lead placement testing apparatus without interruption, even when rotated.

By operating the respective handle 22, 29, the inner lead 3 and the outer lead 5 can be rotated independently. In one example, the two handles 22, 29 and / or the two lead screws 23, 30 have different colours to assist the surgeon in identifying which handle 22, 29 is responsible for the inner lead 3 and which lead screw 23, 30 is responsible for the outer lead 5. Moreover, in one example markings are applied to the circumference one or both of the lead handles 22, 29 in order to assist the surgeon in assessing the rotational position of the lead handles 22, 29.

Another example of the connector 1, which is not shown, differs from the example shown in **Figures 4** to **7** in that it lacks the outer lead clamping fixture. In this case, the lead device 2 is introduced into the housing 13 directly through the opening on the left side of the housing 14 in **Figures 4 to 7****.** In a third example of the connector 1, the outer lead clamping fixture is supplied loose, ie, separate from the connecter without this fixture, and it can be snapped onto the housing 13 with the latch mechanism.

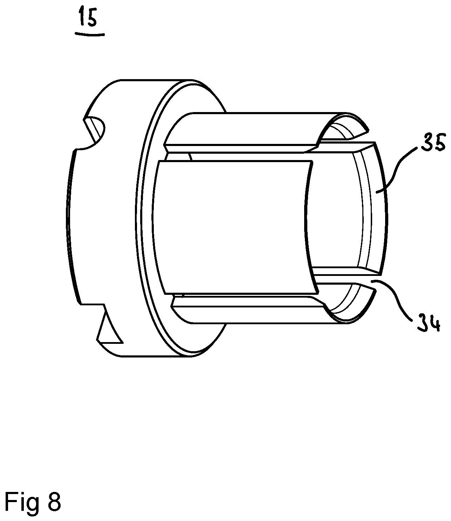

An exemplary inner lead contact 15 in the form of a slotted electrically conducting sleeve is shown in **Figure 8****.** It has four equidistantly arranged slots 34 that extend parallel to each other along the longitudinal axis of the sleeve. The four parts 35 of the sleeve between the slots 34 can resiliently press against the electrode of the inner lead 3 to make electrical contact.

The features as described in the above description, claims and figures can be relevant individually or in any combination to realise the various embodiments of the invention.

### Reference numerals

- 1: connector
- 2: lead device
- 3: inner lead
- 4: inner insulator
- 5: outer lead
- 6: lumen
- 7: inner seal
- 8: electrode
- 9: outer insulator
- 10: outer seal
- 11: lead device cover
- 12: guide wire
- 13: housing
- 14: housing handle
- 15: inner lead contact
- 16: outer lead contact
- 17: strand
- 18: strand
- 19: cable
- 20: collet
- 21: collet sleeve
- 22: inner lead handle
- 23: inner lead screw
- 24: plunger
- 25: central passage
- 26: lid
- 27: collet
- 28: collet sleeve
- 29: outer lead handle
- 30: outer lead screw
- 31: central passage
- 32: lid
- 33: fixation helix
- 34: slots
- 35: parts of sleeve between slots

## Claims

1. A connector (1) for electrically connecting a proximal end of a lead device (2) that is implantable within the body of a patient and comprises a first lead (3, 5), wherein the connector (1) comprises a first lead contact (15, 16) for, directly or indirectly, electrically contacting, at the proximal end of the lead device (2), an electrode of the first lead (3, 5), **characterised in that** the first lead contact (15, 16) comprises a lamella basket.

2. The connector (1) of claim 1, **characterised in that** via the first lead contact (15, 16), the electrode of the first lead (3, 5) is electrically connected with a strand (17, 18) of a cable (19).

3. The connector (1) of any one of the preceding claims, **characterised in that** the first lead contact (15, 16) is attached to a housing (13) of the connector (1) in a way that the first lead contact (15, 16) cannot rotate about its longitudinal axis.

4. The connector (1) of any one of the preceding claims, **characterised in that** it comprises a first lead clamping fixture for clamping the first lead (3, 5).

5. The connector (1) of claim 4, **characterised in that** the first lead clamping fixture is rotatable relatively to the housing (13).

6. The connector (1) of claim 5, **characterised in that** the first lead clamping fixture comprises a first lead handle (22, 29) so that a rotating of the first lead handle (22, 29) relatively to the housing (13) rotates the first lead clamping fixture relatively to the housing (13).

7. The connector (1) of any one of claims 4 or 6, **characterised in that** the first lead clamping fixture comprises a first lead screw (23, 30) for clamping the first lead (3, 5) to the first lead clamping fixture by operating the first lead screw (23, 30).

8. The connector (1) of any one of claims 1 to 7, **characterised in that** the first lead contact (15) is only indirectly electrically contacting the electrode of the first lead (3).

9. The connector (1) of any one of claims 4 to 8, **characterised in that** the first lead clamping fixture is, at least partly, electrically conductive and can electrically contact the electrode of the first lead (3), and the first lead contact (15) can electrically contact the electrode of the first lead (3) via the first lead clamping fixture.

10. The connector (1) according to any one of claims 4 to 9, **characterised in that** the first lead clamping fixture comprises a central passage (25) through which a guide wire (12) of the lead device (2) can pass when the connector (1) is connected to the proximal end of the lead device (2).

11. A connector (1) for electrically connecting a proximal end of a lead device (2) that is implantable within the body of a patient and comprises a first lead (3, 5), wherein the connector (1) comprises a first lead clamping fixture for clamping the first lead (3, 5), **characterised in that** the first lead clamping fixture comprises a collet (20, 27) that can embrace the first lead (3, 5) at a proximal end of the first lead (3, 5), and the first lead clamping fixture moreover comprises a collet sleeve (21, 28) that has an internal taper for cooperating with the collet (20, 27) to secure the collet (20, 27) on the first lead (3, 5) by compressing the collet (20, 27) if the collet (20, 27) is pressed against the collet sleeve's (21, 28) internal taper.

12. The connector (1) of claim 11, **characterised in that** the first lead clamping fixture comprises a first lead handle (22, 29) and a first lead screw (23, 30), wherein the first lead screw (23, 30) is operatively connected with one element of the group comprising the collet (20, 27) and the collet sleeve (21, 28) and comprises a thread, wherein the first lead handle (22, 29) is operatively connected with the other element of the group comprising the collet (20, 27) and the collet sleeve (21, 28) and comprises a matching thread, and wherein the thread of the first lead screw (23, 30) runs in the tread of the first lead handle (22, 29) so that displacement of the first lead screw (23, 30) relatively to the first lead handle (22, 29) when rotating the two relatively to each other can press the collet (20, 27) against the collet sleeve's (21, 28) internal taper.

13. The connector (1) of any one of the preceding claims, characterised that the connector (1) is for electrically connecting a coaxial lead device (2) with an outer lead (5) and an inner lead (2) that extends coaxially insider the outer lead (5), and wherein the first lead is the inner lead (3).

14. The connector (1) according to any one of the preceding claims, **characterised in that** it moreover comprises a second lead contact (15, 16) for, directly or indirectly, electrically contacting, at the proximal end of the lead device (2), an electrode of a second lead (3, 5) of the lead device (2).

15. The connector (1) according to claim 14, **characterised in that** the second lead contact (15, 16) comprises a lamella basket.

16. The connector (1) of claim 14 or 15, **characterised in that** via the second lead contact (15, 16), the electrode of the second lead (3, 5) is electrically connected with a strand (17, 18) of a cable (19).

17. The connector (1) of any one of claims 1 to 16, **characterised in that** it comprises a second lead clamping fixture for clamping the second lead (3, 5).

18. The connector (1) of claim 17, **characterised in that** the second lead clamping fixture comprises a second lead handle (22, 29) so that a rotating of the second lead handle (22, 29) relatively to the housing (13) rotates the second lead clamping fixture relatively to the housing (13).

19. The connector (1) of any one of claims 17 or 18, **characterised in that** the second lead clamping fixture comprises a second lead screw (23, 30) for clamping the second lead (3, 5) to the second lead clamping fixture by operating the second lead screw (23, 30 ).

20. The connector (1) of any one of claims 17 to 19, **characterised in that** the second lead clamping fixture comprises a collet (20, 27) that can embrace the second lead (3, 5) at a proximal end of the second lead (3, 5) and has an external taper, and the second lead clamping fixture moreover comprises a collet sleeve (21, 28) that has an internal taper for cooperating with the collet (20, 27) to secure the collet (20, 27) on the second lead (3, 5) by compressing the collet (20, 27) if the collet is pressed against the collet sleeve's (21, 28) internal taper.

21. The connector (1) according to any one of claims 17 to 20, **characterised in that** the second lead clamping fixture comprises a central passage (31) through which the lead device (2) can pass inside the connector (1) to connect the connector (1) to the proximal end of the lead device (2).

22. The connector (1) of any one of claims 14 to 21, characterised that the connector (1) is for electrically connecting a coaxial lead device (2) with an outer lead (5) and an inner lead (3) that extends coaxially insider the outer lead (5), and wherein the second lead is the outer lead (5).

23. A connector (1) for electrically connecting the proximal end of a lead device (2) that is implantable within the body of a patient and comprises a lead device (2) being a coaxial lead device (2) with an outer lead (5) and an inner lead (3) that extends coaxially inside the outer lead (5) and the inner lead (3) being rotatable relatively to the outer lead (5), wherein the connector (1) comprises a first lead clamping fixture for clamping a first lead, the first lead (3) being one lead from the group of leads comprising the inner lead (3) and the outer lead (5), a first lead handle (22, 29) so that a rotating of the first lead handle (22, 29) relatively to the housing (13) rotates the first lead clamping fixture relatively to the housing (13), a second lead clamping fixture for clamping a second lead, the second lead (5) being the other lead from the group of leads comprising the inner lead (3) and the outer lead (5), and a second lead handle (22, 29) so that a rotating of the second lead handle (22, 29) relatively to the housing (13) rotates the second lead clamping fixture relatively to the housing (13).
